# EUROPEAN PATENT APPLICATION

(11) **EP 4 006 036 A1**
(43) Date of publication of application: **01.06.2022**
(21) Application number: 20854855.2
(22) Date of filing: 20.08.2020
(51) Int. Cl.: C07D 493/14, C07D 493/22, C07D 487/22, C07D 491/22, A61K 31/519, A61K 31/5377, A61P 35/00

(54) **TETRACYCLIC COMPOUND USED AS CDC7 INHIBITOR**

(30) Priority: 20.08.2019 CN 201910769786
(71) Applicant: CHIA TAI TIANQING PHARMACEUTICAL GROUP CO., LTD., Lianyungang, Jiangsu 222062 (CN)
(72) Inventor: LU, Lun, Shanghai 200131 (CN); LI, Gang, Shanghai 200131 (CN); HU, Lihong, Shanghai 200131 (CN); DING, Charles Z., Shanghai 200131 (CN); CHEN, Shuhui, Shanghai 200131 (CN)
(74) Representative: Müller Schupfner & Partner Patent- und Rechtsanwaltspartnerschaft mbB
(86) International application number: PCT/CN2020/110305
(87) International publication number: WO 2021/032170

(57) **Abstract**

A tetracyclic compound as a Cdc7 inhibitor. Specifically disclosed is a compound represented by formula (I), an isomer thereof, or a pharmaceutically acceptable salt thereof. (I)

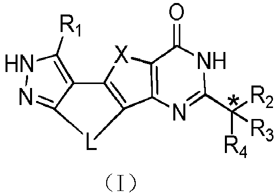

## Description

The present application claims priority to Chinese Patent Application No.

CN201910769786.6 filed on August 20, 2019.

### FIELD OF THE INVENTION

The present invention relates to a new type of tetracyclic compounds as Cdc7 inhibitors, and specifically discloses a compound of formula (I), an isomer thereof or a pharmaceutically acceptable salt thereof.

### BACKGROUND OF THE INVENTION

Cdc7 is a serine/threonine kinase that was first discovered in *Saccharomyces cerevisiae* in 1974, after which scientists also discovered homologous proteins to it in other eukaryotes. Different species of Cdc7 have certain differences in structure but are very similar in functionality. In one aspect, they activate minichromosome maintenance proteins (MCM proteins), an important element of a DNA replication initiator, by phosphorylation to promote the formation of a replication initiation complex, and in another aspect, they can also be used as important regulatory factors of the S-phase checkpoint of a cell cycle for controlling the smooth progress of the cell cycle.

HuCdc7, a homologous protein in human cells to Cdc7, was not discovered by scientists until late 1990s. HuCdc7 is expressed in almost all histiocytes of humans. However, it is found that the abnormal high expression of huCdc7 occurs in various tumor cells of humans, and such abnormal high expression shows high correlation with abnormal proliferation and metastasis of tumors and resistance to chemotherapeutic drugs. Therefore, huCdc7 has become an important marker and target in the current tumor research.

HuCdc7 is expressed in all tissues of human body. It binds to ASK (activator of S phase kinase, also referred to as DBF4) in the nucleus and is thus activated. The activated huCdc7-ASK complex can bind to a chromosome under the action of motif-N on ASK. From the study we can speculate that the process of binding of huCdc7 to ASK and activation is basically as follows: a. In an early G1 phase, huCdc7 enters the nucleus under the action of importin B and bind to a chromosome; b. In a late G1 phase, ASK enters the nucleus under the action of a nuclear localization signal; c. ASK binds to the chromosome under the mediation of motif-N and to huCdc7 under the action of motif-M and motif-C, thus activating huCdc7. Upon formation of huCdc7-ASK complex in the nucleus, it activates, by phosphorylation, multiple members of the minichromosome maintenance protein complex (MCM) family that bind to the chromosome, such as MCM2, MCM4 and MCM6, and in particular, MCM2 is highly phosphorylated. MCMs are an important component of a helicase in an initiation complex in a cell cycle.

The activity of huCdc7 is also regulated by phosphorylation of huCdc7 by cell cycle factors. The activation of huCdc7 may require not only the binding to the auxiliary protein ASK, but also the regulation by phosphorylation of huCdc7 by some cytokines such as Cdk (cyclin-dependent kinase). Cdk2-Cyclin E and Cdk2-Cyclin A in the Cdk family are able to phosphorylate huCdc7 at Thr-376 and other sites, and the phosphorylation at these sites plays an important role in activating huCdc7. It can be seen that the activity of huCdc7 changes with the cell cycle due to the regulation by ASK and cytokines, and huCdc7 is strictly regulated by the cell cycle while facilitating the progress of the cell cycle.

When replication damage occurs, huCdc7 can phosphorylate MCM2 at a number of amino acid sites apart from the activation site, and meanwhile the auxiliary subunit ASK of huCdc7 phosphorylates MCM2 at Ser41, which ultimately causes MCM2 to fall from the chromosome and thus prevents the S phase in the cell cycle to reduce damage to the cell. Montagnoli et al. surprisingly found that Ser108 in a number of amino acid sites phosphorylated by huCdc7 is also a phosphorylation site for ATR, an important factor in the DNA damage response. It is speculated that huCdc7 and ATR may act synergistically in response to DNA damage caused by replication. Thus, it can be seen that when DNA replication damage occurs, phosphorylation of MCM2 at amino acid sites such as Ser108 by huCdc7 and phosphorylation of MCM2 at Ser41 by ASK play an important role in inactivating MCM2 to maintain cell survival. When DNA damage occurs during replication in the cell cycle, huCdc7 can not only inhibit the initiation of replication to maintain cell survival, but also participate in the DNA damage signaling pathway to facilitate cell cycle arrest and DNA repair. Kim et al. found that when DNA replication damage occurs during replication in the S phase of the cell cycle, huCdc7 activates Claspin by phosphorylation to further phosphorylate Chk1 at Ser317 and Ser345, thereby activating the ATR/Chk1 pathway in response to the DNA replication damage in the cell cycle to arrest mitosis and repair DNA and thus to maintain cell survival. Chk1 is an important checkpoint protein when DNA damage occurs during replication. When DNA damage occurs during replication, Chk1 is highly expressed and activated, which can cause phosphorylation of Cdc25 at Ser216 to inhibit Cdc25 activity and thus MPF activity, resulting in cell cycle arrest in the damaged cell. Active Chk1 can also facilitate the aggregation of some DNA damage signaling proteins and DNA repair proteins at the DNA damage sites to repair DNA damage and maintain cell survival. Another study has shown that Chk1 can also phosphorylate ASK at multiple amino acid sites in the DNA damage response in the cell cycle. The above studies suggest that huCdc7 may be involved in the ATR/Chk1 signaling pathway in the DNA damage checkpoint response in such a manner: a. When DNA replication damage occurs in the cell cycle, single-stranded DNA activates ATR → ATR further activates Chk1 → Chk1 phosphorylates ASK at multiple sites to facilitate change in the functionality of huCdc7 bound to the ASK, that is, huCdc7 starts to respond to the DNA replication damage instead of promoting initiation of DNA replication. b. HuCdc7, in one aspect, phosphorylates MCM2 at multiple sites apart from the activation site with the aid of ATR to inactivate MCM2 and thus prevent a DNA replication initiation complex from forming, and in another aspect, further activates the ATR/Chk1 signaling pathway by phosphorylating Claspin in response to the DNA damage. It can be seen that huCdc7 is both an effector and amplification factor for the ATR/Chk1 signaling pathway in DNA replication damage response.

HuCdc7 is expressed at a constant level in a normal cell cycle, and is in a state of dynamic equilibrium due to the regulation by several factors and auxiliary proteins in the cell cycle. HuCdc7 is abnormally expressed and over-activated in tumor cells due to disturbances of the cell cycle. Hess et al. found that due to over-expression of huCdc7 in various tumor cells, the over-expressed huCdc7 may promote over-activation of MCM2, an important marker for tumor cells, and thus the abnormal proliferation of tumor cells. Besides, they also found that huCdc7 is highly expressed in all metastatic tumor cells, suggesting that the abnormal high expression of huCdc7 may be closely associated with the metastasis of tumor cells. Nambiar et al. have recently found that the auxiliary protein ASK of huCdc7 is also highly expressed in multiple cutaneous melanoma cell lines, which further enhances the activity of huCdc7 in tumor cells. In addition, the abnormal high expression and activation of huCdc7 play a key role in resistance to chemotherapeutic drugs for tumor cells. Tenca et al. found that huCdc7 is extensively expressed with high activity after treating tumor cells with chemotherapeutic drugs Hu and etoposide, and it was noted in the research that huCdc7 inhibits the activity of the two drugs and thus reduces the damage to tumor cells by phosphorylating MCM2 and MCM4 at multiple amino acid sites, which is specifically characterized by the following: a. Phosphorylation of MCM2 at multiple sites, such as Ser41 and Ser108, can further inhibit assembly of an initiation complex; b. Phosphorylation of MCM4 at multiple sites can promote inaccurate localization of Cdc45 on chromosomes, eventually disrupting DNA replication initiation. In addition, Tenca et al. also found that huCdc7 can delay extension of a replication fork by regulating the precipitation of chromatin assembly factor 1 and histone at the replication fork and reduce the damage to tumor cells to maintain tumor cell survival. Another study has demonstrated that huCdc7 can also protect tumor cells by phosphorylating Claspin to further activate the ATR/Chk1 pathway and thus to arrest mitosis and repair damaged DNA. HuCdc7 can be inhibited to promote the apoptosis of tumor cells since huCdc7 plays an important role in promoting the proliferation, metastasis and drug resistance of the tumor cells. In order to inhibit the continuous proliferation and metastasis of tumor cells, Montagnoli et al. introduced siRNA into tumor cells to inhibit the high expression of huCdc7. Experimental results show that the phosphorylation level of MCM2 at the activation site is greatly reduced, the DNA replication initiation of the tumor cells is inhibited, and the growth of the tumor cells is slowed down. In addition, after huCdc7 in p53-deficient tumor cells is inhibited by siRNA and the tumor cells are treated with the chemotherapeutic drug Hu, they found that the phosphorylation level of the important downstream factor Chk1 at Ser 345 in the DNA replication damage checkpoint ATR/Chk1 pathway is greatly reduced, leading to the failure of the tumor cells to normally respond to DNA damage and the aggregation of the otherwise dispersed DNA in the S phase, and thereby resulting in disturbances of the DNA replication of the tumor cells in the S phase and apoptosis of the tumor cells through a non-p53 pathway. Im et al. further found that in p53-deficient tumor cells in which huCdc7 is specifically inhibited by siRNA, the DNA replication damage checkpoint fails to respond, and ATR can promote apoptosis of tumor cells by activating p38 MAPK. When huCdc7 is down-regulated and DNA replication damage occurs in normal cells in a cell cycle, normally functioning p53 can be activated and further up-regulate and activate p21 to arrest the cell cycle and repair damaged cells to maintain normal cell survival. In addition, Kim et al. found that after huCdc7 is inhibited by siRNA, the reduced phosphorylation level of MCM4 at N-terminus leads to the failure of Cdc45 to be located on a chromosome, and thus no replication initiation complex is formed, which also inhibits tumor proliferation to some extent. Therefore, as long as the activity of huCdc7 in tumor cells can be effectively inhibited, the growth of the tumor cells can be effectively inhibited and the apoptosis of the tumor cells can be promoted without causing damage to normal cells.

TAK-931, as a Cdc7 inhibitor, features high inhibitory activity against Cdc7 (CDC7/DBF4 IC₅₀ 2.59 nM) and anti-proliferative activity against the colo205 cell line with high expression of Cdc7 (COLO205 IC₅₀ 85.51 nM). It demonstrates excellent inhibitory activity against tumors in 25 PDX models and is in phase II clinical trials at present. However, due to its high clearance rate and short half-life *in vivo*, there is a clinical need for developing a new generation of Cdc7 inhibitor capable of being stably metabolized.

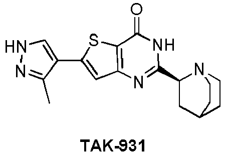

### SUMMARY

The present invention provides a compound of formula (I), an isomer thereof or a pharmaceutically acceptable salt thereof, 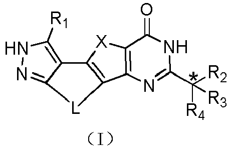 wherein,
the carbon atom with "^{∗}" can be a chiral carbon atom present in a form of a single (R) or (S) enantiomer or in a form enriched in one enantiomer;
X is selected from the group consisting of O, NH and NCH₃;
L is selected from the group consisting of -CH₂-CH₂-CH₂-, -CH₂-O-CH₂-, -CH₂-S-CH₂-, -CH₂-NH-CH₂-, -NH-CH₂-CH₂-, -S-CH₂-CH₂- and -O-CH₂-CH₂-;
R₁ is selected from the group consisting of H, halogen, CN, C₁₋₆ alkyl, C₃₋₆ cycloalkyl, phenyl, and 5-6 membered heteroaryl, wherein the C₁₋₆ alkyl, C₃₋₆ cycloalkyl, phenyl, and 5-6 membered heteroaryl are each independently optionally substituted with 1, 2 or 3 Rₐ, the 5-6 membered heteroaryl containing 1, 2 or 3 heteroatoms or heteroatom groups each independently selected from the group consisting of O, S, N and NH;
R₂ is selected from R_{b}, R₃ is selected from NH₂, and R₄ is selected from H;
alternatively, R₂ is selected from R_{c}, and R₃ and R₄ are joined to form a ring A optionally substituted with 1, 2 or 3 Rₑ, wherein the ring A is selected from the group consisting of C₆₋₁₄ aryl, 5-14 membered heteroaryl, 5-12 membered heterocycloalkenyl and 4-14 membered heterocycloalkyl each independently containing 1, 2 or 3 heteroatoms or heteroatom groups independently selected from the group consisting of O, S, N and NR_{d};
Rₐ is each independently selected from the group consisting of F, Cl, Br, I, OH, CN, NH₂, CH₃ and 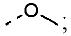
R_{b} is selected from the group consisting of H and C₁₋₆ alkyl, the C₁₋₆ alkyl being optionally substituted with 1, 2 or 3 R;
R_{c} is selected from the group consisting of H, F, Cl, Br, I and C₁₋₃ alkyl;
R_{d} is selected from the group consisting of H and C₁₋₄ alkyl;
R is selected from the group consisting of -OCH₃, -OCH₂CH₃, -O-CH(CH₃)₂, cyclopropyl, cyclopentyl, phenyl, pyrazolyl, pyridinyl, NH₂, -NHCH₃ and -N(CH₃)₂;
Rₑ is selected from the group consisting of F, Cl, Br, I, OH, CN, COOH, NH₂, -NHCH₃, -N(CH₃)₂, CH₃, CH₂CH₃, CF₃, -OCH₃, -OCH₂CH₃, -O-CH(CH₃)₂, -C(=O)OCH₃, -C(=O)CH₃ and -C(=O)CH₂CH₃.

In some embodiments of the present invention, R₁ is selected from the group consisting of H, F, Cl, Br, I, CN, CH₃, CH₂CH₃, cyclopropyl, phenyl and pyridinyl, wherein the CH₃, CH₂CH₃, cyclopropyl, phenyl and pyridinyl are each independently optionally substituted with 1, 2 or 3 Rₐ, and the other variables are as defined herein.

In some embodiments of the present invention, R₁ is selected from the group consisting of H, F, Cl, Br, I, CN, CH₃, CH₂CH₃, CF₃, cyclopropyl, phenyl and pyridinyl, and the other variables are as defined herein.

In some embodiments of the present invention, R₁ is selected from H, and the other variables are as defined herein.

In some embodiments of the present invention, R_{b} is selected from the group consisting of H, methyl, ethyl, isopropyl, propyl, butyl and isobutyl, and the other variables are as defined herein.

In some embodiments of the present invention, R_{c} is selected from the group consisting of H, methyl, ethyl and F, and the other variables are as defined herein.

In some embodiments of the present invention, R_{d} is selected from the group consisting of H, methyl, ethyl, propyl, isopropyl and *n*-butyl, and the other variables are as defined herein.

In some embodiments of the present invention, the ring A is selected from 5-9 membered heterocycloalkyl containing a heteroatom or heteroatom group selected from the group consisting of N and NR_{d}, and the other variables are as defined herein.

In some embodiments of the present invention, the ring A is selected from the group consisting of 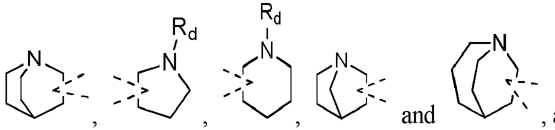 and the other variables are as defined herein.

In some embodiments of the present invention, the structural unit 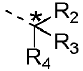 is selected from the group consisting of 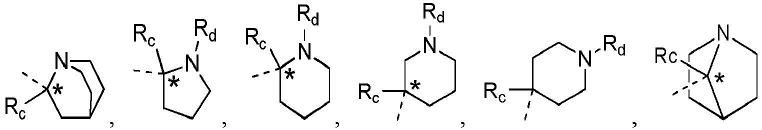 and 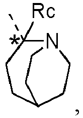 and the other variables are as defined herein.

In some embodiments of the present invention, the structural unit 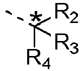 is selected from the group consisting of 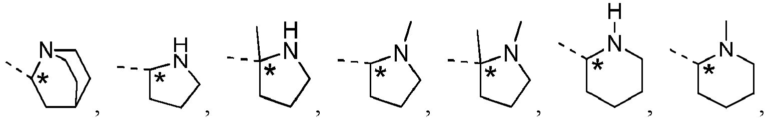 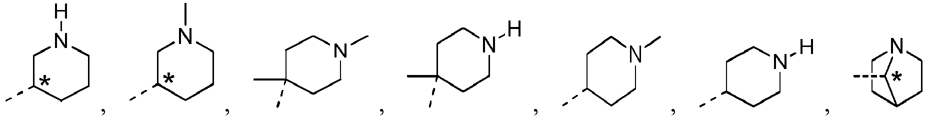 and 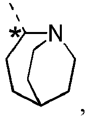 and the other variables are as defined herein.

In some embodiments of the present invention, the structural unit 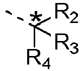 is selected from 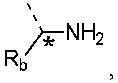 and the other variables are as defined herein.

In some embodiments of the present invention, L is selected from -CH₂-CH₂-CH₂-, and the other variables are as defined herein.

In some embodiments of the present invention, the compound is selected from: 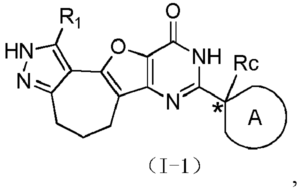 wherein the carbon atom with "^{∗}" can be a chiral carbon atom present in a form of a single (R) or (*S*) enantiomer or in a form enriched in one enantiomer, and R₁ and the ring A are as defined herein.
Still some other embodiments of the present invention are derived from any combination of the variables described above.

The present invention further provides a compound of the following formulas, an isomer thereof or a pharmaceutically acceptable salt thereof:

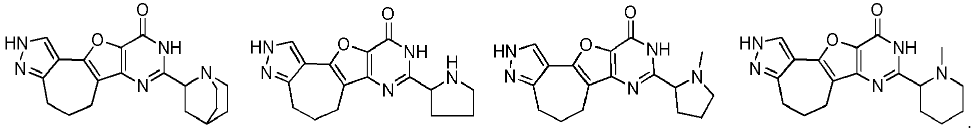

In some embodiments of the present invention, the compound, the isomer thereof or the pharmaceutically acceptable salt thereof is selected from the group consisting of:

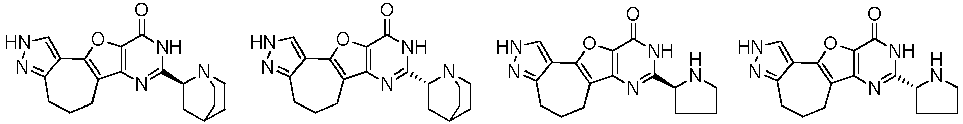

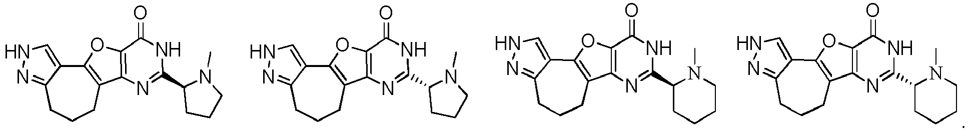

The present invention further provides a pharmaceutical composition comprising a therapeutically effective amount of the compound, the isomer thereof or the pharmaceutically acceptable salt thereof and a pharmaceutically acceptable carrier.

The present invention also provides use of the compound, the isomer thereof or the pharmaceutically acceptable salt thereof or the pharmaceutical composition for preparing a medicament for treating a tumor.

In some embodiments of the present invention, the medicament for treating a tumor refers to a medicament for treating colorectal cancer or pancreatic cancer.

### Technical Effects

The compound disclosed herein, as a Cdc7 inhibitor, has a wide application prospect in treating tumors since it demonstrates a unique inhibitory effect on tumors in cancer treatment and has no toxic side effects on normal cells. Therefore, further intensive research on the Cdc7 kinase and inhibitors thereof is expected to pave a new way for clinically treating tumors. The compound disclosed herein is expected to become a new medicament with better therapeutic effects and lower toxic side effects compared to similar products.

### Definitions and Description

Unless otherwise stated, the following terms and phrases used herein are intended to have the following meanings. A particular term or phrase, unless otherwise specifically defined, should not be considered as uncertain or unclear, but construed according to its common meaning. When referring to a trade name, it is intended to refer to its corresponding commercial product or its active ingredient.

The term "pharmaceutically acceptable" is used herein for those compounds, materials, compositions and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound disclosed herein, which is prepared from the compound having particular substituents disclosed herein and a relatively nontoxic acid or base. When the compound disclosed herein contains a relatively acidic functional group, a base addition salt can be given by contacting such a compound with a sufficient amount of a base in a pure solution or a suitable inert solvent. Pharmaceutically acceptable base addition salts include sodium, potassium, calcium, ammonium, organic amine, or magnesium salts, or similar salts. When the compound disclosed herein contains a relatively basic functional group, an acid addition salt can be obtained by contacting such a compound with a sufficient amount of an acid in a pure solution or a suitable inert solvent. Examples of pharmaceutically acceptable acid addition salts include salts derived from inorganic acids, such as hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid and phosphorous acid; and salts derived from organic acids, such as acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid and methanesulfonic acid. Also included are salts of amino acids (e.g., arginine) and salts of organic acids such as glucuronic acid. Certain specific compounds disclosed herein contain both basic and acidic functional groups that allow the compounds to be converted into either base or acid addition salts.

The pharmaceutically acceptable salts disclosed herein can be synthesized from a parent compound having an acidic or basic group by conventional chemical methods. In general, such salts are prepared by the following method: reacting the free acid or base form of the compound with a stoichiometric amount of the appropriate base or acid in water or an organic solvent or a mixture thereof.

The compound disclosed herein may demonstrate a specific geometric isomerism or stereoisomerism. All such compounds are contemplated herein, including *cis* and *trans* isomers, (-)- and (+)-enantiomers, (R)- and (*S*)-enantiomers, diastereoisomers, (D)-isomers, (L)-isomers, and racemic mixtures and other mixtures thereof, such as an enantiomer or diastereomer enriched mixture, all of which are encompassed within the scope of the present invention. Substituents such as alkyl may have an additional asymmetric carbon atom. All these isomers and mixtures thereof are encompassed within the scope of the present invention.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely.

Unless otherwise stated, the term "diastereoisomer" refers to stereoisomers whose molecules have two or more chiral centers and are not mirror images of each other.

Unless otherwise stated, "(+)" stands for dextrorotation, "(-)" stands for levorotation, and "(±)" stands for racemization.

Unless otherwise stated, the absolute configuration of a stereogenic center is represented by a wedged solid bond ( 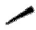 ) and a wedged dashed bond ( 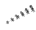 ), and the relative configuration of a stereogenic center is represented by a straight solid bond ( 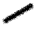 ) and a straight dashed bond ( 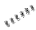 ). A wavy line ( 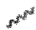 ) represents a wedged solid bond ( 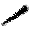 ) or a wedged dashed bond ( 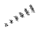 ), or a wavy line ( 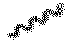 ) represents a straight solid bond ( 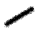 ) or a straight dashed bond ( 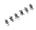 ).

Unless otherwise stated, the term "enriched in one isomer", "isomer enriched", "enriched in one enantiomer", or "enantiomer enriched" means that the content of one of the isomers or enantiomers is less than 100% and more than or equal to 60%, 70%, 80%, 90%, 95%, 96%, 97%, 98%, 99%, 99.5%, 99.6%, 99.7%, 99.8% or 99.9%.

Unless otherwise stated, the term "isomeric excess" or "enantiomeric excess" refers to the difference between the relative percentages of two isomers or enantiomers. For example, if the content of one isomer or enantiomer is 90% and the content of the other isomer or enantiomer is 10%, the isomeric or enantiomeric excess (ee) is 80%.

Optically active (*R*)- and (*S*)-isomers and *D* and *L* isomers can be prepared by chiral synthesis or chiral reagents or other conventional techniques. If one enantiomer of a certain compound disclosed herein is to be obtained, the desired pure enantiomer can be prepared by asymmetric synthesis or derivatization using a chiral additive, wherein the resulting diastereoisomeric mixture is separated and the auxiliary group is cleaved. Alternatively, when the molecule contains a basic functional group (such as amino) or an acidic functional group (such as carboxyl), the compound reacts with an appropriate optically active acid or base to form a salt of the diastereoisomer, which is then subjected to diastereoisomeric resolution through conventional methods in the art to give the pure enantiomer. Furthermore, the enantiomer and the diastereoisomer are generally isolated through chromatography using a chiral stationary phase, optionally in combination with chemical derivatization (e.g., carbamate generated from amines). The compound disclosed herein may contain an unnatural proportion of atomic isotope at one or more of the atoms that constitute the compound. For example, the compound may be labeled with a radioisotope, such as tritium (³H), iodine-125 (¹²⁵I), or C-14 (¹⁴C). For another example, hydrogen can be substituted with deuterium to form a deuterated drug, and the bond formed by deuterium and carbon is firmer than that formed by common hydrogen and carbon. Compared with an un-deuterated drug, the deuterated drug has the advantages of reduced toxic side effects, increased stability, enhanced efficacy, prolonged biological half-life and the like. All isotopic variations of the compound disclosed herein, whether radioactive or not, are encompassed within the scope of the present invention.

The term "substituted" means that one or more hydrogen atoms on a specific atom are substituted with substituents which may include deuterium and hydrogen variants, as long as the valence of the specific atom is normal and the substituted compound is stable. When the substituent is an oxygen (i.e., =O), it means that two hydrogen atoms are substituted. Substitution with oxygen does not occur on aromatic groups. The term "optionally substituted" means that an atom can be substituted with a substituent or not. Unless otherwise specified, the type and number of the substituent may be arbitrary as long as being chemically achievable.

When any variable (e.g., R) occurs more than once in the constitution or structure of a compound, the variable is independently defined in each case. Thus, for example, if a group is substituted with 0-2 R, the group can be optionally substituted with up to two R, and the definition of R in each case is independent. Furthermore, a combination of a substituent and/or a variant thereof is permissible only if the combination can result in a stable compound.

When the number of a linking group is 0, for example, -(CRR)o-, it means that the linking group is a single bond.

When the number of a substituent is 0, it means that there is no such a substituent in a structure. For example, -A-(R)o means that the structure is actually -A.

When a substituent is absent, it means that there is no such a substituent in a structure. For example, when X is absent in A-X, it means that the structure is actually A.

When one of variables is selected from a single bond, it means that the two groups are linked directly. For example, in A-L-Z, when L represents a single bond, it means that the structure is actually A-Z.

When a bond of a substituent can be cross-linked to two or more atoms on a ring, the substituent can be bonded to any atom on the ring. For example, the structural unit 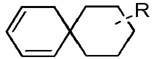 or 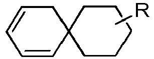 represents that the substitution of substituent R may occur in any one position on cyclohexyl or cyclohexadienyl. When it is not specified by which atom the listed substituent is linked to the group to be substituted, the substituent can be bonded via its any atom. For example, pyridinyl as a substituent can be linked to the group to be substituted via any carbon atom on the pyridine ring.

When the listed linking group does not indicate the direction for linking, the direction for linking is arbitrary. For example, when the linking group L contained in 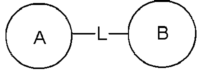 is -M-W-, -M-W- can either link ring A and ring B in a direction same as left-to-right reading order to form 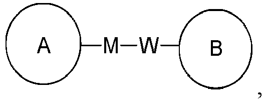 or link ring A and ring B in a direction opposite to the left-to-right reading order to form 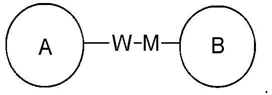 A combination of the linking group, a substituent and/or a variant thereof is permissible only if the combination can result in a stable compound.

Unless otherwise specified, when a group has one or more linkable sites, any one or more of the sites of the group may be linked to other groups by chemical bonds. When there is no designated linking mode for a chemical bond and H atoms are present at a linkable site, the number of the H atoms at the linkable site is correspondingly reduced based on the number of the linked chemical bonds, and a group with a corresponding valence number is thus formed. The chemical bond that links the site to another group may be represented by a straight solid bond ( 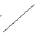 ), a straight dashed bond ( 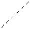 ), or a wavy line ( 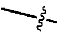 ). For example, the straight solid bond in -OCH₃ refers to being linked to another group via the oxygen atom in the group; the straight dashed bond in 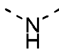 refers to being linked to another group via two ends of the nitrogen atom in the group; the wavy line in 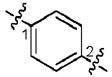 refers to being linked to another group via the carbon atoms at positions 1 and 2 in the phenyl group; 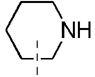 means that any linkable site on the piperidinyl can be linked to another group via 1 bond, and at least 4 linking modes 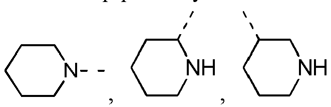 and 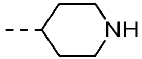 are possible; even if -N- is linked to an H atom, 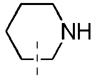 includes the linking mode of 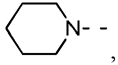 except that when 1 bond is linked to a site, the number of H at that site is correspondingly reduced by 1 and a monovalent piperidinyl is thus formed.

Unless otherwise specified, the number of atoms on a ring is generally defined as the member number of the ring. For example, "5-7 membered ring" refers to a "ring" on which 5 to 7 atoms are arranged in a circle.

Unless otherwise specified, the term "C₁₋₆ alkyl" refers to a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆, and C₅ alkyl, etc., and may be monovalent (e.g., methyl), divalent (e.g., methylene) or polyvalent (e.g., methenyl). Examples of C₁₋₆ alkyl include, but are not limited to, methyl (Me), ethyl (Et), propyl (including *n*-propyl and isopropyl), butyl (including *n*-butyl, isobutyl, s-butyl, and *t*-butyl), pentyl (including *n*-pentyl, isopentyl, and neopentyl), hexyl, and the like.

Unless otherwise specified, the term "C₁₋₄ alkyl" refers to a linear or branched saturated hydrocarbon group consisting of 1 to 4 carbon atoms. The C₁₋₄ alkyl includes, but is not limited to, C₁₋₂, C₁₋₃ and C₂₋₃ alkyl, and the like, and may be monovalent (e.g., methyl), divalent (e.g., methylene) or polyvalent (e.g., methenyl). Examples of C₁₋₄ alkyl include, but are not limited to, methyl (Me), ethyl (Et), propyl (including *n*-propyl and isopropyl), butyl (including *n*-butyl, isobutyl, s-butyl and *t*-butyl), and the like.

Unless otherwise specified, the term "C₁₋₃ alkyl" refers to a linear or branched saturated hydrocarbon group consisting of 1 to 3 carbon atoms. The C₁₋₃ alkyl includes, but is not limited to, C₁₋₂ and C₂₋₃ alkyl, etc., and may be monovalent (e.g., methyl), divalent (e.g., methylene) or polyvalent (e.g., methenyl). Examples of C₁₋₃ alkyl include, but are not limited to, methyl (Me), ethyl (Et), propyl (including *n*-propyl and isopropyl), and the like.

Unless otherwise specified, the term "halo" or "halogen", by itself or as part of another substituent, refers to a fluorine, chlorine, bromine or iodine atom.

Unless otherwise specified, "C₃₋₆ cycloalkyl" refers to a saturated cyclic hydrocarbon group consisting of 3 to 6 carbon atoms, which is a monocyclic and bicyclic ring system, wherein the carbon atoms may optionally be oxidized (i.e., C=O). The C₃₋₆ cycloalkyl includes C₃₋₅, C₄₋₅, C₅₋₆ cycloalkyl and the like, and may be monovalent, divalent or polyvalent. Examples of C₃₋₆ cycloalkyl include, but are not limited to, cyclopropyl, cyclobutyl, cyclopentyl, cyclohexyl and the like.

Unless otherwise specified, the term "4-14 membered heterocycloalkyl", by itself or in combination with other terms, refers to a saturated cyclic group consisting of 4 to 14 ring atoms, of which 1, 2, 3 or 4 ring atoms are heteroatoms independently selected from the group consisting of O, S and N, with the remaining being carbon atoms. The nitrogen atom is optionally quaternized, and the carbon atoms and the nitrogen and sulfur heteroatoms can be optionally oxidized (i.e., C=O, NO and S(O)ₚ, wherein p is 1 or 2). This includes monocyclic, bicyclic and tricyclic systems, wherein the bicyclic and tricyclic systems include spirocyclic, fused and bridged rings. Furthermore, with respect to the "4-14 membered heterocycloalkyl", a heteroatom may occupy the position where the heterocycloalkyl is linked to the rest of the molecule. The 4-14 membered heterocycloalkyl includes 4-12 membered, 5-10 membered, 5-9 membered, 3-10 membered, 3-8 membered, 3-6 membered, 3-5 membered, 4-6 membered, 5-6 membered, 4 membered, 5 membered and 6 membered heterocycloalkyl and the like. Examples of 4-14 membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, 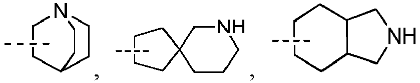 or the like.

Unless otherwise specified, the term "5-9 membered heterocycloalkyl", by itself or in combination with other terms, refers to a saturated cyclic group consisting of 5 to 9 ring atoms, of which 1, 2, 3 or 4 ring atoms are heteroatoms independently selected from the group consisting of O, S and N, with the remaining being carbon atoms. The nitrogen atom is optionally quaternized, and the carbon atoms and the nitrogen and sulfur heteroatoms can be optionally oxidized (i.e., C=O, NO and S(O)ₚ, wherein p is 1 or 2). This includes monocyclic and bicyclic systems, wherein the bicyclic system includes spirocyclic, fused, and bridged rings. Furthermore, with respect to the "5-9 membered heterocycloalkyl", a heteroatom may occupy the position where the heterocycloalkyl is linked to the rest of the molecule. The 5-9 membered heterocycloalkyl includes 9 membered, 8 membered, 7 membered, 6 membered, 5 membered heterocycloalkyl, and the like. Examples of 5-9 membered heterocycloalkyl include, but are not limited to, pyrrolidinyl, pyrazolidinyl, imidazolidinyl, tetrahydrothienyl (including tetrahydrothien-2-yl, tetrahydrothien-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl, dioxepanyl, 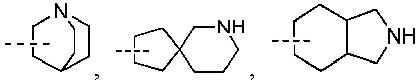 or the like.

Unless otherwise specified, the term "5-12 membered heterocycloalkenyl", by itself or in combination with other terms, refers to a partially unsaturated cyclic group consisting of 5 to 12 ring atoms containing at least one carbon-carbon double bond, of which 1, 2, 3 or 4 ring atoms are heteroatoms independently selected from the group consisting of O, S and N, with the remaining being carbon atoms. The nitrogen atom is optionally quaternized, and the carbon atoms and the nitrogen and sulfur heteroatoms can be optionally oxidized (i.e., C=O, NO and S(O)ₚ, wherein p is 1 or 2). This includes monocyclic, bicyclic and tricyclic systems, wherein the bicyclic and tricyclic systems include spirocyclic, fused and bridged rings, and any ring of these systems is nonaromatic. Furthermore, with respect to the "5-12 membered heterocycloalkenyl", a heteroatom may occupy the position where the heterocycloalkenyl is linked to the rest of the molecule. The 5-12 membered heterocycloalkenyl includes 5-10 membered, 5-8 membered, 5-6 membered, 4-5 membered, 4 membered, 5 membered, 6 membered heterocycloalkenyl and the like. Examples of 5-12 membered heterocycloalkenyl include, but are not limited to,

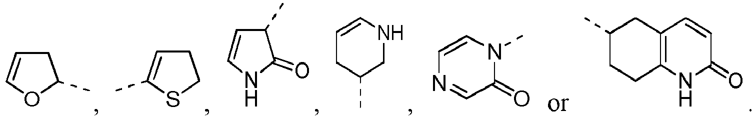

Unless otherwise specified, the terms "C₆₋₁₄ aromatic ring" and "C₆₋₁₄ aryl" in the present invention are used interchangeably. The term "C₆₋₁₄ aromatic ring" or "C₆₋₁₄ aryl" refers to a cyclic hydrocarbon group consisting of 6 to 14 carbon atoms and having a conjugated π-electron system. The group may be a monocyclic, fused bicyclic or fused tricyclic system, where the rings are aromatic. It may be monovalent, divalent or polyvalent, and the C₆₋₁₄ aryl includes C₆₋₁₀, C₆₋₉, C₆₋₈, C₁₂, C₁₄, C₁₀ and C₆ aryl and the like. Examples of C₆₋₁₄ aryl include, but are not limited to, phenyl, naphthyl (including 1-naphthyl, 2-naphthyl, etc.) and anthryl.

Unless otherwise specified, the term "5-14 membered heteroaromatic ring" and "5-14 membered heteroaryl" herein are used interchangeably. The term "5-14 membered heteroaryl" refers to a cyclic group consisting of 5 to 14 ring atoms and having a conjugated π-electron system, of which 1, 2, 3 or 4 ring atoms are heteroatoms independently selected from the group consisting of O, S and N, with the remaining being carbon atoms. The nitrogen atom is optionally quaternized, and the carbon atoms and the nitrogen and sulfur heteroatoms may optionally be oxidized (i.e., C=O, NO and S(O)ₚ, wherein p is 1 or 2). It can be a monocyclic, fused bicyclic or fused tricyclic system, wherein the rings are aromatic. The 5-14 membered heteroaryl can be linked to the rest of the molecule via a heteroatom or a carbon atom. The 5-14 membered heteroaryl includes 5-12 membered, 5-10 membered, 5-8 membered, 5-7 membered, 5-6 membered, 5 membered, 6 membered heteroaryl and the like. Examples of the 5-12 membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, pyrimidinyl (including 2-pyrimidinyl, 4-pyrimidinyl, etc.), benzothiazolyl (including 5-benzothiazolyl, etc.), purinyl, benzimidazolyl (including 2-benzimidazolyl, etc.), benzoxazolyl, indolyl (including 5-indolyl, etc.), isoquinolinyl (including 1-isoquinolinyl, 5-isoquinolinyl, etc.), quinoxalinyl (including 2-quinoxalinyl, 5-quinoxalinyl, etc.), quinolyl (including 3-quinolyl, 6-quinolyl, etc.) or benzoisoquinolin.

Unless otherwise specified, the terms "5-6 membered heteroaromatic ring" and "5-6 membered heteroaryl" are used interchangeably herein. The term "5-6 membered heteroaryl" refers to a monocyclic group consisting of 5 to 6 ring atoms and having a conjugated π-electron system, in which 1, 2, 3 or 4 ring atoms are heteroatoms independently selected from the group consisting of O, S, and N, while the others are carbon atoms. The nitrogen atom is optionally quaternized, and the carbon atoms and the nitrogen and sulfur heteroatoms are optionally oxidized (i.e., C=O, NO and S(O)ₚ, where p is 1 or 2). The 5-6 membered heteroaryl can be linked to the rest of the molecule via a heteroatom or a carbon atom. The 5-6 membered heteroaryl includes 5 membered and 6 membered heteroaryl. Examples of the 5-6 membered heteroaryl include, but are not limited to, pyrrolyl (including *N*-pyrrolyl, 2-pyrrolyl, 3-pyrrolyl, etc.), pyrazolyl (including 2-pyrazolyl, 3-pyrazolyl, etc.), imidazolyl (including *N*-imidazolyl, 2-imidazolyl, 4-imidazolyl, 5-imidazolyl, etc.), oxazolyl (including 2-oxazolyl, 4-oxazolyl, 5-oxazolyl, etc.), triazolyl (including 1*H*-1,2,3-triazolyl, 2*H*-1,2,3-triazolyl, 1*H*-1,2,4-triazolyl, 4*H*-1,2,4-triazolyl, etc.), tetrazolyl, isoxazolyl (including 3-isoxazolyl, 4-isoxazolyl, 5-isoxazolyl, etc.), thiazolyl (including 2-thiazolyl, 4-thiazolyl, 5-thiazolyl, etc.), furanyl (including 2-furanyl, 3-furanyl, etc.), thienyl (including 2-thienyl, 3-thienyl, etc.), pyridinyl (including 2-pyridinyl, 3-pyridinyl, 4-pyridinyl, etc.), pyrazinyl, or pyrimidinyl (including 2-pyrimidinyl, 4-pyrimidinyl, etc.).

The compound disclosed herein can be prepared by a variety of synthetic methods well known to those skilled in the art, including the specific examples listed below, examples formed by combinations thereof with other chemical synthetic methods, and equivalents thereof known to those skilled in the art. Preferred examples include, but are not limited to, the examples of the present invention.

The solvent used in the present invention can be commercially available.

The following abbreviations are used in the present invention: aq for water; HATU for O-(7-azabenzotriazol-1-yl)-*N*,*N*,*N*',*N*'-tetramethyluronium hexafluorophosphate; EDC for *N*-(3-dimethylaminopropyl)-*N*'-ethylcarbodiimide hydrochloride; m-CPBA for 3-chloroperoxybenzoic acid; eq for equivalent; CDI for carbonyldiimidazole; DCM for dichloromethane; PE for petroleum ether; DIAD for diisopropyl azodicarboxylate; DMF for *N*,*N*-dimethylformamide; DMSO for dimethyl sulfoxide; EtOAc for ethyl acetate; EtOH for ethanol; MeOH for methanol; Cbz for benzyloxycarbonyl, an amine protecting group; BOC for *t*-butoxycarbonyl, an amine protecting group; HOAc for acetic acid; NaCNBH₃ for sodium cyanoborohydride; r.t. for room temperature; O/N for overnight; THF for tetrahydrofuran; Boc₂O for di-*tert*-butyl dicarbonate; TFA for trifluoroacetic acid; DIPEA for diisopropylethylamine; SOCl₂ for thionyl chloride; CS₂ for carbon disulphide; TsOH for *p*-toluenesulfonic acid; NFSI for *N*-fluoro-*N*-(phenylsulfonyl)benzenesulfonamide; NCS for 1-chloropyrrolidine-2,5-dione; *n*-Bu₄NF for tetrabutylammonium fluoride; iPrOH for 2-propanol; mp for melting point; and LDA for lithium diisopropylamide.

Compounds are named according to conventional nomenclature rules in the art or using ChemDraw^{®} software, and supplier's catalog names are given for commercially available compounds.

### DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various changes and modifications can be made to the specific examples without departing from the spirit and scope of the present invention.

### Example 1. Compounds 1-1 and 1-2

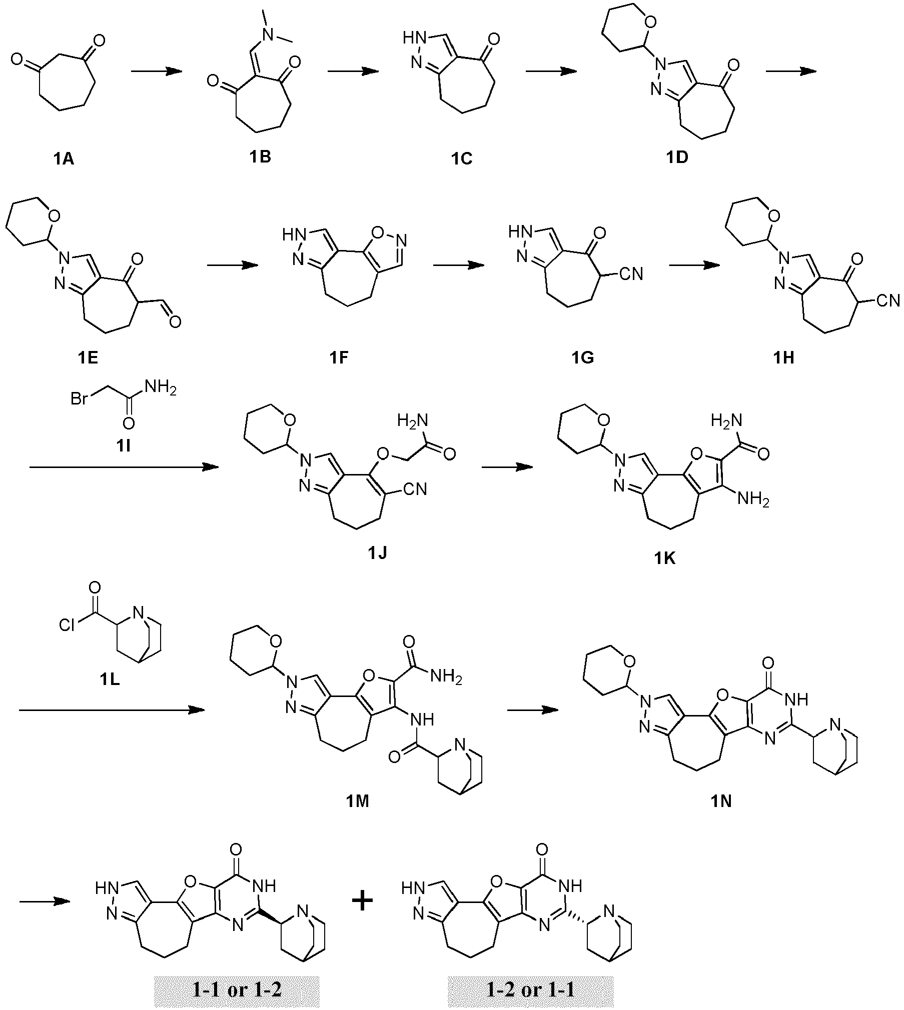

### Preparation of compound 1B:

Compound 1A (20 g, 158.54 mmol) was stirred in *N*,*N*-dimethylformamide dimethyl acetal (53.82 g, 451.66 mmol) at 100 °C for 3 h. The reaction system was concentrated to dryness under reduced pressure to give compound 1B without purification, which was directly used in the next step. LCMS (ESI) m/z: 182 (M+1).

### Preparation of compound 1C:

Hydrazine hydrate (6.96 g, 139.05 mmol) was added to a solution of 1B (28 g, 154.5 mmol) in methanol (80 mL) at 0 °C. The reaction system was heated to 90 °C and stirred for 2 h. The reaction system was concentrated to dryness under reduced pressure. The residue was stirred in 150 mL of a mixture (ethyl acetate/petroleum ether = 1/9, v/v) for 10 min, and the resulting mixture was filtered to give solid compound 1C. LCMS (ESI) m/z: 151 (M+1).

### Preparation of compound 1D:

Compound 1C (3.0 g, 19.98 mmol), 3,4-dihydro-2*H*-pyran (2.52 g, 29.96 mmol) and trifluoroacetic acid (228 mg, 2.0 µmol) were stirred in tetrahydrofuran (50 mL) at 50 °C for 5 h. The reaction system was cooled to room temperature. Water (20 mL) was added, followed by ethyl acetate (50 mL × 2) for extraction. The organic phase was washed with saturated brine (10 mL × 2), dried over anhydrous sodium sulfate and filtered. The filtrate was concentrated by rotary evaporation. The residue was purified by column chromatography (1000 mesh silica gel, petroleum ether/ethyl acetate = 100/1 to 3/1) to give compound 1D. LCMS (ESI) m/z: 235 (M+1).¹H NMR (deuterated methanol, 400 MHz) δppm 8.21 (s, 1H), 5.33-5.42 (m, 1H), 4.02-4.11 (m, 1H), 3.70-3.77 (m, 1H), 2.95-3.02 (m, 2H), 2.68-2.74 (m, 2H), 1.94-2.05 (m, 6H), 1.57-1.82 (m, 4H).

### Preparation of compound 1E:

To a solution of compound 1D (3.5 g, 14.94 mmol) in tetrahydrofuran (50 mL) was added sodium hydride (1.19 g, 29.88 mmol, 60%) at 20 °C under nitrogen atmosphere. The reaction system was stirred at 20 °C for 0.5 h. Ethyl formate (1.66 g, 22.41 mmol) was then added, and the reaction was continued for another 2 h. The reaction system was quenched with saturated ammonium chloride solution (20 mL) and extracted with ethyl acetate (80 mL × 2). The organic phase was washed with saturated brine (30 mL), dried over anhydrous sodium sulfate and filtered. The filtrate was concentrated by rotary evaporation to give compound 1E. LCMS (ESI) m/z: 263 (M+1).

### Preparation of compound 1F:

To a solution of compound 1E (3.9 g, 14.87 mmol) in ethanol (15 mL) was added hydroxylamine hydrochloride (1.08 g, 14.87 mmol) at 25 °C. The reaction system was stirred at 90 °C for 2 h and filtered to give compound IF. LCMS (ESI) m/z: 176 (M+1). ¹H NMR (deuterated methanol, 400 MHz) δppm 8.39-8.47 (m, 1H), 8.25-8.32 (m, 1H), 3.12-3.23 (m, 2H), 2.85-2.94 (m, 2H), 2.06-2.17 (m, 2H).

### Preparation of compound 1G:

To a solution of compound IF (2.5 g, 14.27 mmol) in ethanol (20 mL) was added sodium methoxide (1.54 g, 28.54 mmol) at 20 °C under nitrogen atmosphere. The reaction system was stirred at 75 °C for 2 h. The reaction system was cooled to room temperature, quenched with saturated ammonium chloride solution (5 mL) and extracted with ethyl acetate (20 mL × 2). The organic phase was washed with saturated brine (10 mL), dried over anhydrous sodium sulfate and filtered. The filtrate was concentrated by rotary evaporation to give compound 1G. LCMS (ESI) m/z: 176 (M+1).

### Preparation of compound 1H:

Compound 1G (1.7 g, 9.7 mmol), 3,4-dihydro-2*H*-pyran (1.06 g, 12.62 mmol) and trifluoroacetic acid (111 mg, 970.39 µmol) were stirred in tetrahydrofuran (50 mL) at 60 °C for 3 h. The reaction system was cooled to room temperature and concentrated by rotary evaporation. The residue was purified by column chromatography (1000 mesh silica gel, petroleum ether/ethyl acetate = 10/1 to 1/1) to give **compound** 1H. LCMS (ESI) m/z: 260 (M+1).

### Preparation of compound 1J:

Compound 1H (0.5 g, 1.93 mmol), compound 1I (399 mg, 2.89 mmol) and potassium carbonate (799 mg, 5.78 mmol) were stirred in *N*,*N*-dimethylformamide (10 mL) at 100 °C for 3 h. The reaction system was cooled to room temperature and concentrated by rotary evaporation. The residue was purified by column chromatography (1000 mesh silica gel, petroleum ether/ethyl acetate = 10/1 to 1/1) to give compound 1J. LCMS (ESI) m/z: 317 (M+1).

### Preparation of compound 1K

Sodium (29 mg, 1.25 mmol) was added to ethanol (9 mL) at 20 °C. After sodium completely disappeared, compound 1J (0.2 g, 623.47 µmol) was added. The reaction system was then refluxed for 5 h. The reaction system was concentrated to dryness under reduced pressure. The residue was purified by reversed-phase flash chromatography (Agela Technologies, C18, 20-35 µm, 0.1% aqueous formic acid solution/acetonitrile) to give compound 1K. LCMS (ESI) m/z: 317 (M+1).¹H NMR (deuterated methanol, 400 MHz) δ ppm 7.97 (s, 1H), 5.27 - 5.16 (m, 1H), 3.96 - 3.89 (m, 1H), 3.66 - 3.58 (m, 1H), 2.89 - 2.82 (m, 2H), 2.56 - 2.49 (m, 2H), 2.06 - 1.92 (m, 4H), 1.72 - 1.44 (m, 4H).

### Preparation of compound 1M:

To a solution of compound 1K (181 mg, 1.04 mmol) and compound 1L (110 mg, 347.71 µmol) in dichloromethane (10 mL) was added diisopropylethylamine (180 mg, 1.39 mmol) at 20 °C under nitrogen atmosphere. The reaction system was stirred at 20 °C for 5 h. The reaction system was concentrated to dryness to give crude compound 1M, which was directly used in the next step. LCMS (ESI) m/z: 454 (M+1).

### Preparation of compound 1N:

Compound 1M (150.7 mg, 347.71 µmol) was dissolved in a solvent mixture of methanol (10 mL) and water (10 mL) at 20 °C, and sodium hydroxide (139.09 mg, 3.48 mmol) was then added. The reaction system was warmed to 70 °C and stirred for 30 min. Methanol was evaporated. Water (10 mL) was added, followed by ethyl acetate (50 mL × 2) for extraction. The organic phase was washed with saturated brine (10 mL × 2), dried over anhydrous sodium sulfate and filtered. The filtrate was concentrated by rotary evaporation to give crude compound IN, which was directly used in the next step. LCMS (ESI) m/z: 436 (M+1).

### Preparation of examples 1-1 and 1-2:

To a solution of compound IN (0.075 g, 172.21 µmol) in dichloromethane (3 mL) was dropwise added trifluoroacetic acid (2 mL, 27.01 mmol) at 25 °C. The reaction system was stirred at room temperature for 1 h. The reaction system was concentrated. The residue was purified by reversed-phase flash chromatography (Agilent, C18 reversed-phase column, 20-35 µm, 0.1% formic acid aqueous solution/acetonitrile). The resulting product was separated by SFC (Daicel CHIRALCEL OJ-H (250 mm × 30 mm, 5 µm); mobile phase: carbon dioxide as phase A, methanol containing 0.1% ammonium hydroxide as phase B; elution gradient: 30%-30% phase B, 3.95 min) to give two fractions, which were re-purified by preparative high performance liquid chromatography (column: Phenomenex Synergi C18 150 × 25 × 10 µm; mobile phase: 0.05% diluted hydrochloric acid as phase A, acetonitrile as phase B; elution gradient: 11%-31% phase B, 11 min) to give compound 1-1 and compound 1-2.

Determined using the SFC analytical method below, the retention times of compound 1-1 and compound 1-2 were 2.089 min and 1.919 min, respectively.

SFC analytical method:
Column: Chiralcel OD-3 50 × 4.6 mm I.D., 3 µm; mobile phase: carbon dioxide as phase A, ethanol containing 0.05% diethylamine as phase B; gradient elution: 5%-40% phase B; flow rate: 3 mL/min; wavelength: 220 nm; column temperature: 35 °C; back pressure: 100 Bar.
Compound 1-1: LCMS (ESI) m/z: 352 (M+1).¹H NMR (400 MHz, deuterated methanol) δppm 8.50 - 7.90 (m, 1H), 4.06 - 3.95 (m, 1H), 3.43 (br s, 4H), 3.18 - 3.09 (m, 1H), 3.04 - 2.86 (m, 2H), 2.58 - 2.47 (m, 1H), 2.38 - 2.28 (m, 1H), 2.20 - 1.80 (m, 8H).

Compound 1-2: LCMS (ESI) m/z: 352 (M+1). ¹H NMR (400 MHz, deuterated methanol) δppm 8.53 - 8.00 (m, 1H), 4.21 - 3.78 (m, 1H), 3.72 - 3.37 (m, 3H), 3.18 - 2.77 (m, 4H), 2.61 - 2.41 (m, 1H), 2.35 - 2.23 (m, 1H), 2.17 - 1.85 (m, 8H).

### Example 2. Compounds 2-1 and 2-2

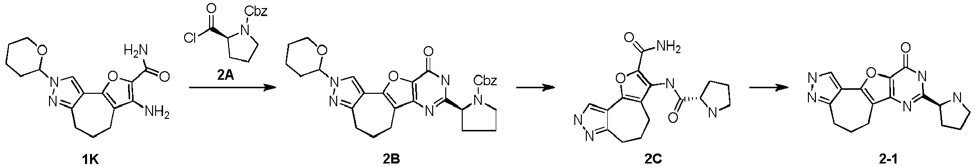

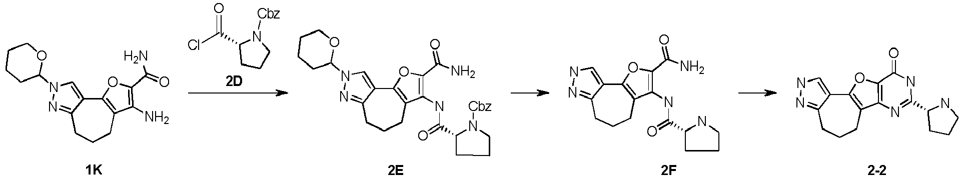

### Preparation of compound 2B:

To a solution of compound 1K (500 mg, 1.57 mmol) and compound 2A (1.07 g, 4.0 µmol) in dichloromethane (10 mL) was added diisopropylethylamine (608 mg, 4.71 mmol) at 20 °C under nitrogen atmosphere. The reaction system was stirred at 20 °C for 5 h. The reaction system was concentrated to dryness. The residue was purified by reversed-phase flash chromatography (Agilent, C18 reversed-phase column, 20-35 µm, 0.1% formic acid aqueous solution/acetonitrile) to give compound 2B. LCMS (ESI) m/z: 530 (M+1).

### Preparation of compound 2C:

To compound 2B (0.4 g, 755.31 µmol) was added dropwise a 35% solution of hydrobromic acid in acetic acid (5 mL) at room temperature. The reaction system was stirred at 20 °C for 1 h. The reaction system was concentrated to give crude compound 2C, which was directly used in the next step. LCMS (ESI) m/z: 330 (M+1).

### Preparation of compound 2-1:

Compound 2C (238 mg, 722.63 µmol) was dissolved in methanol (10 mL) at room temperature, and sodium hydroxide (289 mg, 7.23 mmol) was added. The reaction system was warmed to 70 °C and stirred for 30 min. Methanol was evaporated. The residue was purified by preparative high performance liquid chromatography (column: Phenomenex Synergi C18 150 × 25 × 10 µm; mobile phase: 0.05% diluted hydrochloric acid as phase A, acetonitrile as phase B; elution gradient: 6%-26% phase B, 9 min) to give compound 2-1. LCMS (ESI) m/z: 312 (M+1).¹H NMR (400 MHz, deuterated methanol) δ ppm 8.24 - 8.17 (m, 1H), 4.82 - 4.75 (m, 1H), 3.71 - 3.61 (m, 1H), 3.55 - 3.46 (m, 1H), 3.20 - 3.13 (m, 2H), 3.11 - 3.03 (m, 2H), 2.70 - 2.55 (m, 1H), 2.24 - 2.17 (m, 2H), 2.17 - 2.17 (m, 1H), 2.17 - 2.10 (m, 2H).

### Preparation of compound 2E:

To a solution of compound 1K (300 mg, 941.05 µmol) and compound 2D (0.504 g, 1.88 mmol) in dichloromethane (10 mL) was added diisopropylethylamine (365 mg, 2.82 mmol) at 20 °C under nitrogen atmosphere. The reaction system was stirred at 20 °C for 5 h. The reaction system was concentrated to dryness to give crude compound 2E. LCMS (ESI) m/z: 548 (M+1).

### Preparation of compound 2F:

To compound 2E (0.5 g, 913.07 µmol) was added dropwise a 30% solution of hydrobromic acid in acetic acid (5 mL) at room temperature. The reaction system was stirred at 20 °C for 1 h. The reaction system was concentrated to give crude compound 2F, which was directly used in the next step. LCMS (ESI) m/z: 330 (M+1).

### Preparation of compound 2-2:

The compound was prepared as described for example compound 2-1. LCMS (ESI) m/z: 312 (M+1).¹H NMR (400 MHz, deuterated methanol) δ ppm 8.25 - 8.15 (m, 1H), 4.83 - 4.75 (m, 1H), 3.71 - 3.61 (m, 1H), 3.54 - 3.46 (m, 1H), 3.21 - 3.13 (m, 2H), 3.11 - 3.03 (m, 2H), 2.69 - 2.57 (m, 1H), 2.24 - 2.12 (m, 5H).

Determined using the SFC analytical method below, the retention times of compound 1-1 and compound 1-2 were 0.991 min and 1.298 min, respectively.

SFC analytical method:
Column: Chiralpak AD-3 50 × 4.6 mm I.D., 3 µm; mobile phase: carbon dioxide as phase A, ethanol containing 0.05% diethylamine as phase B; gradient elution: isocratic elution with 40% phase B; flow rate: 3 mL/min; wavelength: 220 nm; column temperature: 35 °C; back pressure: 100 Bar.

### Example 3. Compounds 3-1 and 3-2

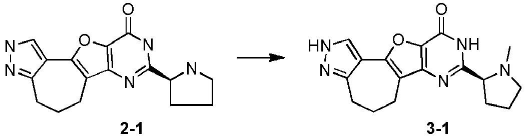

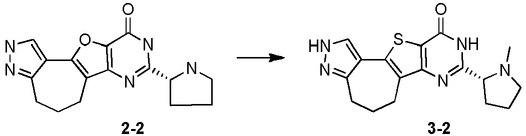

### Preparation of compound 3-1:

Compound 2-1(50.00 mg, 160.14 µmol), 37% aqueous formaldehyde solution (22 µL, 800.69 µmol) and sodium cyanoborohydride (30 mg, 480.41 µmol) were stirred in methanol (5 mL) at 20 °C for 1 h. The reaction system was concentrated. The residue was purified by preparative high performance liquid chromatography (column: Phenomenex Synergi C18 150 × 25 × 10 µm; mobile phase: 0.05% aqueous hydrochloric acid solution as phase A, acetonitrile as phase B; elution gradient: 6%-26% acetonitrile, time: 12 min) to give compound 3-1. LCMS (ESI)m/z: 326 (M+1).¹H NMR (400 MHz, deuterated methanol) δ ppm 8.30 - 8.19 (m, 1H), 4.68 - 4.57 (m, 1H), 4.03 - 3.90 (m, 1H), 3.51 - 3.36 (m, 1H), 3.22 - 3.15 (m, 2H), 3.13 (s, 3H), 3.11 - 3.05 (m, 2H), 2.85 - 2.73 (m, 1H), 2.40 - 2.27 (m, 1H), 2.26 - 2.12 (m, 4H).

### Preparation of compound 3-2:

The compound was prepared as described for compound 3-1. LCMS (ESI)m/z: 326 (M+1).¹H NMR (400 MHz, deuterated methanol) δ ppm 8.30 (s, 1H), 4.67 - 4.56 (m, 1H), 4.03 - 3.90 (m, 1H), 3.46 - 3.39 (m, 1H), 3.23 - 3.17 (m, 2H), 3.12 (s, 3H), 3.12 - 3.07 (m, 2H), 2.87 - 2.73 (m, 1H), 2.43 - 2.28 (m, 1H), 2.27 - 2.14 (m, 4H).

Determined using the SFC analytical method below, the retention times of compound 1-1 and compound 1-2 were 1.190 min and 1.116 min, respectively.

SFC analytical method:
Column: Chiralcel OJ-3 50 × 4.6 mm I.D., 3 µm; mobile phase: carbon dioxide as phase A, methanol containing 0.05% diethylamine as phase B; gradient elution: 5%-40% phase B; flow rate: 3 mL/min; wavelength: 220 nm; column temperature: 35 °C; back pressure: 100 Bar.

### Example 4. Compound 4

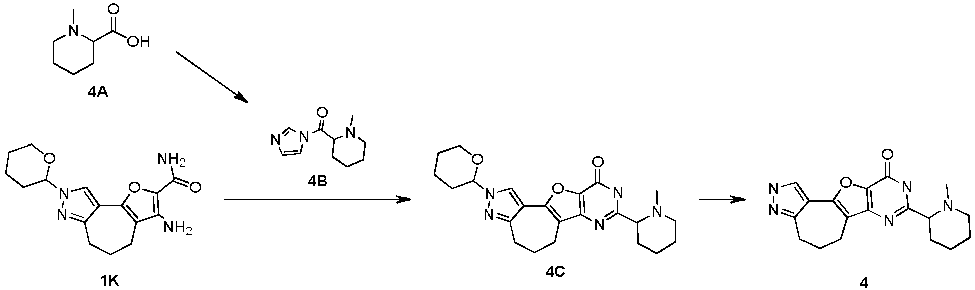

### Preparation of compound 4B:

To a solution of compound 4A (0.375 g, 2.62 mmol) and diisopropylamine (1.02 g, 7.86 mmol) in dichloromethane (5 mL) was added 1,1-carbonyldiimidazole (0.425 g, 2.62 mmol) at 20 °C under nitrogen atmosphere. The reaction system was concentrated to dryness under reduced pressure to give compound 4B.

### Preparation of compound 4C:

To a solution of compound 1K (300 mg, 869.83 µmol) and compound 4B (500 mg, 2590 µmol) in N,N-dimethylformamide (10 mL) was added diisopropylethylamine (450 mg, 3480 µmol) at 20 °C. The reaction system was stirred at 100 °C for 12 h and then at 120 °C for 36 h. The reaction system was concentrated to dryness to give crude compound 4C. LCMS (ESI) m/z: 424 (M+1).

### Preparation of compound 4:

To a solution of compound 4C (0.357 g, 842.96 µmol) in dichloromethane (3 mL) was dropwise added trifluoroacetic acid (2 mL, 27.01 mmol) at room temperature. The reaction system was stirred at 20 °C for 2 h. The reaction system was concentrated. The residue was purified by preparative high performance liquid chromatography (column: Phenomenex Synergi C18 150 × 25 × 10 µm; mobile phase: 0.05% diluted hydrochloric acid as phase A, acetonitrile as phase B; elution gradient: 0%-25% acetonitrile, time: 10 min) to give compound 4. LCMS (ESI) m/z: 340 (M+1).¹H NMR (400 MHz, deuterated methanol) δ ppm 8.05 - 8.01 (m, 1H), 4.23 - 4.16 (m, 1H), 3.74 - 3.66 (m, 1H), 3.16 - 3.11 (m, 2H), 3.07 - 3.03 (m, 2H), 2.93 (s, 3H), 2.37 - 2.29 (m, 1H), 2.18 - 2.09 (m, 2H), 2.06 (m, 5H), 1.81 - 1.71 (m, 1H).

### Experimental Example 1. Detection of Inhibitory Effect of Compounds Against Activity of Cdc7/DBF4 Kinase

### Materials:

Cdc7/DBF4 kinase detection kit purchased from Promega; and
Nivo multi-marker analyzer (PerkinElmer).

### Method:

An enzyme, a substrate, adenosine triphosphate and an inhibitor were diluted with the kinase buffer in the kit.

A test compound was serially 5-fold diluted to an 8th concentration, i.e., from 10 µM to 0.13 nM, with the DMSO concentration being 5%, and the duplicate well experiment was set up. To a microplate were added 1 µL of inhibitors of various concentration gradients, 2 µL of CDC7/DBF4 kinase (6.25 ng), 2 µL of a mixture of substrate and ATP (10 µM adenosine triphosphate, 0.2 µg/µL substrate), and the final concentration gradient of the compound was diluted from 2 µM to 0.025 nM. The reaction system was left reacting at 25 °C for 60 min. After the reaction was completed, 5 µL of ADP-Glo reagent was added to each well, and the reaction was continued at 25 °C for 40 min. After the reaction was completed, 10 µL of the kinase detection reagent was added to each well. After 30 min of reaction at 25 °C, the chemiluminescence was read using a multi-marker analyzer, with an integration time of 0.5 s.

### Data analysis:

The original data were converted to inhibition rate using the equation **(Sample** - **Min)/(Max** - **Min)** × **100%,** and the IC₅₀ value was then curve fitted using four parameters (obtained from the "log(inhibitor) vs. response-Variableslope" model in GraphPadPrism). Table 1 provides the inhibitory activity of the compounds disclosed herein against Cdc7/DBF4 kinase.

**Results:** see Table 1.

**Table 1. Inhibitory activity of the compounds disclosed herein against Cdc7/DBF4 kinase**

| **Compound number** | **Cdc7/DBF4 IC₅₀ (nmol)** | **Compound number** | **Cdc7/DBF4 IC₅₀ (nmol)** |
|---|---|---|---|
| Compound 1-1 | 1.49 | Compound 2-2 | 1.41 |
| Compound 1-2 | 2.8 | Compound 3-1 | 0.6 |
| Compound 2-1 | 0.95 | Compound 3-2 | 1.97 |

| | | | |
|---|---|---|---|
| **Conclusion:** The compounds disclosed herein exhibit good inhibitory activity against Cdc7/DBF4 kinase. | | | |

### Experimental Example 2. Detection of Inhibitory Effect of Compounds Against Activity of Colo205 Cells

### Materials:

1640 medium; fetal bovine serum; penicillin/streptomycin antibiotics purchased from Wisent;
CellTiter-Glo (chemiluminescence detection reagent for cell viability) reagent purchased from Promega;
COLO205 cell line purchased from Wuhan Procell Life Science&Technology Co., Ltd; and Nivo multi-marker analyzer (PerkinElmer).

### Method:

COLO205 cells were plated on to white 96-well plates by adding 80 µL of cell suspension (containing 3000 COLO205 cells) to each well. The cell plate was incubated in a CO₂ incubator overnight.

A test **compound** was serially 3-fold diluted to an 8th concentration, i.e., from 2 mM to 920 nM, and the duplicate well experiment was set up. 78 µL of medium was added to an intermediate plate, 2 µL of the serially diluted compound was transferred to corresponding wells of the intermediate plate, and after mixing, the mixture was transferred to the cell plate at 20 µL per well. The concentration of the compound transferred to the cell plate ranged from 10 µM to 4.57 nM. The cell plate was incubated in a CO₂ incubator for 3 days. Another cell plate was read for signal values on the day of compound addition, and these values were used as the maximum values (the Max value in the equation below) in data analysis. The chemiluminescence detection reagent for cell viability was added to this cell plate at 25 µL per well and the luminescence signals were stabilized by incubation at room temperature for 10 min. Readings were taken using a multi-marker analyzer.

The chemiluminescence detection reagent for cell viability was added to the cell plate at 25 µL per well and the luminescence signals were stabilized by incubation at room temperature for 10 min. Readings were taken using a multi-marker analyzer.

### Data analysis:

The original data were converted to inhibition rate using the equation **(Sample** - **Min)/(Max** - **Min)** × **100%,** and the IC₅₀ value was then curve fitted using four parameters (obtained from the "log(inhibitor) vs. response-Variableslope" model in GraphPadPrism). Table 2 provides the inhibitory activity of the compounds disclosed herein against COLO205 cell proliferation.

**Results:** see Table 2.

**Table 2. Inhibitory activity of the compounds disclosed herein against COLO205 cell proliferation**

| **Compound number** | **Colo205 IC₅₀ (nmol)** |
|---|---|
| Compound 1-2 | 51.72 |

| | |
|---|---|
| **Conclusion:** The compounds disclosed herein exhibit good inhibitory activity against COLO205 cells. | |

## Claims

1. A compound of formula (I), an isomer thereof or a pharmaceutically acceptable salt thereof, 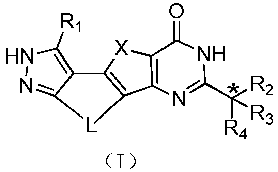 wherein,
the carbon atom with "^{∗}" can be a chiral carbon atom present in a form of a single (R) or (S) enantiomer or in a form enriched in one enantiomer;
X is selected from the group consisting of O, NH and NCH₃;
L is selected from the group consisting of -CH₂-CH₂-CH₂-, -CH₂-O-CH₂-, -CH₂-S-CH₂-, -CH₂-NH-CH₂-, -NH-CH₂-CH₂-, -S-CH₂-CH₂- and -O-CH₂-CH₂-;
R₁ is selected from the group consisting of H, halogen, CN, C₁₋₆ alkyl, C₃₋₆ cycloalkyl, phenyl, and 5-6 membered heteroaryl, wherein the C₁₋₆ alkyl, C₃₋₆ cycloalkyl, phenyl, and 5-6 membered heteroaryl are each independently optionally substituted with 1, 2 or 3 Rₐ, the 5-6 membered heteroaryl containing 1, 2 or 3 heteroatoms or heteroatom groups each independently selected from the group consisting of O, S, N and NH;
R₂ is selected from R_{b}, R₃ is selected from NH₂, and R₄ is selected from H;
alternatively, R₂ is selected from R_{c}, and R₃ and R₄ are joined to form a ring A optionally substituted with 1, 2 or 3 Rₑ, wherein the ring A is selected from the group consisting of C₆₋₁₄ aryl, 5-14 membered heteroaryl, 5-12 membered heterocycloalkenyl and 4-14 membered heterocycloalkyl each independently containing 1, 2 or 3 heteroatoms or heteroatom groups independently selected from the group consisting of O, S, N and NR_{d};
Rₐ is each independently selected from the group consisting of F, Cl, Br, I, OH, CN, NH₂, CH₃ and 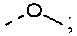
R_{b} is selected from the group consisting of H and C₁₋₆ alkyl, the C₁₋₆ alkyl being optionally substituted with 1, 2 or 3 R;
R_{c} is selected from the group consisting of H, F, Cl, Br, I and C₁₋₃ alkyl;
R_{d} is selected from the group consisting of H and C₁₋₄ alkyl;
R is selected from the group consisting of -OCH₃, -OCH₂CH₃, -O-CH(CH₃)₂, cyclopropyl, cyclopentyl, phenyl, pyrazolyl, pyridinyl, NH₂, -NHCH₃ and -N(CH₃)₂;
Rₑ is selected from the group consisting of F, Cl, Br, I, OH, CN, COOH, NH₂, -NHCH₃, -N(CH₃)₂, CH₃, CH₂CH₃, CF₃, -OCH₃, -OCH₂CH₃, -O-CH(CH₃)₂, -C(=O)OCH₃, -C(=O)CH₃ and -C(=O)CH₂CH₃.

2. The compound, the isomer thereof or the pharmaceutically acceptable salt thereof according to claim 1, wherein R₁ is selected from the group consisting of H, F, Cl, Br, I, CN, CH₃, CH₂CH₃, cyclopropyl, phenyl and pyridinyl, wherein the CH₃, CH₂CH₃, cyclopropyl, phenyl and pyridinyl are each independently optionally substituted with 1, 2 or 3 Rₐ.

3. The compound, the isomer thereof or the pharmaceutically acceptable salt thereof according to claim 2, wherein R₁ is selected from the group consisting of H, F, Cl, Br, I, CN, CH₃, CH₂CH₃, CF₃, cyclopropyl, phenyl and pyridinyl.

4. The compound, the isomer thereof or the pharmaceutically acceptable salt thereof according to claim 3, wherein R₁ is selected from H.

5. The compound, the isomer thereof or the pharmaceutically acceptable salt thereof according to any of claims 1 to 4, wherein R_{b} is selected from the group consisting of H, methyl, ethyl, isopropyl, propyl, butyl and isobutyl.

6. The compound, the isomer thereof or the pharmaceutically acceptable salt thereof according to any of claims 1 to 4, wherein R_{c} is selected from the group consisting of H, methyl, ethyl and F.

7. The compound, the isomer thereof or the pharmaceutically acceptable salt thereof according to any of claims 1 to 4, wherein R_{d} is selected from the group consisting of H, methyl, ethyl, propyl, isopropyl and *n*-butyl.

8. The compound, the isomer thereof or the pharmaceutically acceptable salt thereof according to any of claims 1 to 4, wherein the ring A is selected from 5-9 membered heterocycloalkyl containing 1 heteroatom or heteroatom group selected from the group consisting of N and NR_{d}.

9. The compound, the isomer thereof or the pharmaceutically acceptable salt thereof according to claim 8, wherein the ring A is selected from the group consisting of

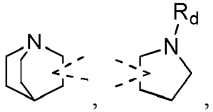

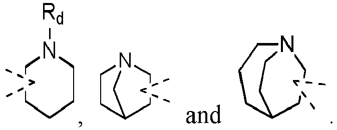

10. The compound, the isomer thereof or the pharmaceutically acceptable salt thereof according to claim 9, wherein the structural unit 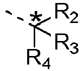 is selected from the group consisting of

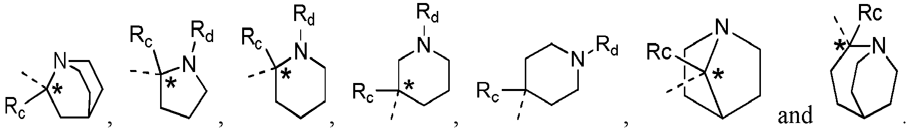

11. The compound, the isomer thereof or the pharmaceutically acceptable salt thereof according to claim 10, wherein the structural unit 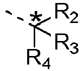 is selected from the group consisting of

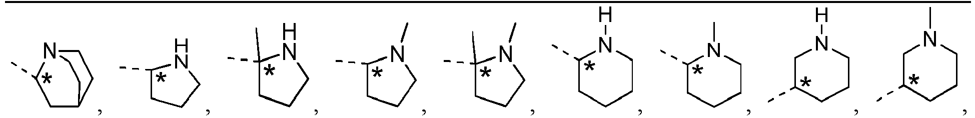

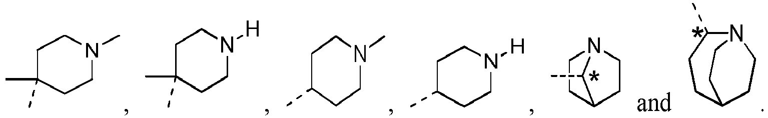

12. The compound, the isomer thereof or the pharmaceutically acceptable salt thereof according to any of claims 1 to 4, wherein the structural unit 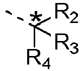 is selected from

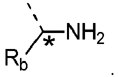

13. The compound, the isomer thereof or the pharmaceutically acceptable salt thereof according to any of claims 1 to 4, wherein L is selected from -CH₂-CH₂-CH₂-.

14. The compound, the isomer thereof or the pharmaceutically acceptable salt thereof according to any of claims 1 to 4 and 8 to 9, wherein the compound is selected from 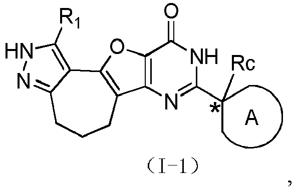 wherein the carbon atom with "^{∗}" can be a chiral carbon atom present in a form of a single (R) or (*S*) enantiomer or in a form enriched in one enantiomer; R₁ is as defined in any of claims 1 to 4; the ring A is as defined in any of claims 1 and 8 to 9.

15. A compound of the following formulas, an isomer thereof or a pharmaceutically acceptable salt thereof, 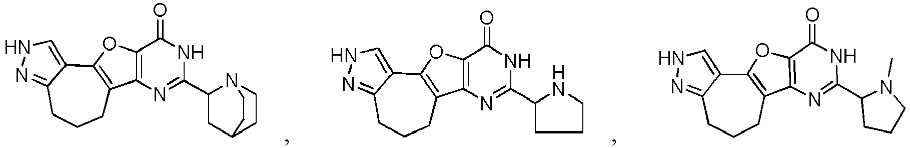 and

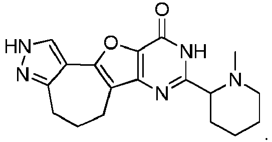

16. The compound, the isomer thereof or the pharmaceutically acceptable salt thereof according to claim 15, selected from the group consisting of

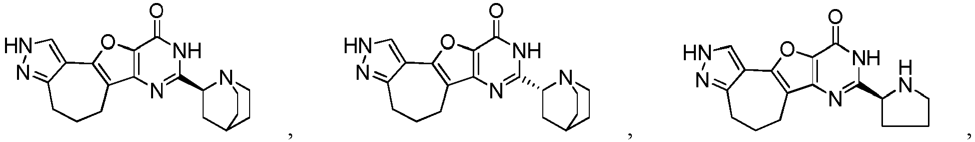

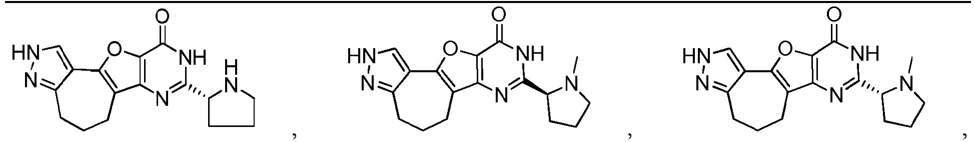

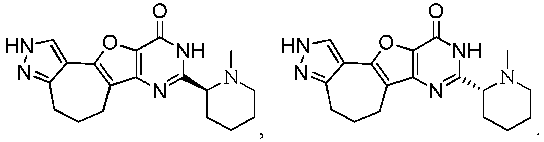

17. A pharmaceutical composition comprising a therapeutically effective amount of the compound or the pharmaceutically acceptable salt thereof according to any of claims 1 to 16 and a pharmaceutically acceptable carrier.

18. Use of the compound or the pharmaceutically acceptable salt thereof according to any one of claims 1 to 16, or the pharmaceutical composition according to claim 17 for preparing a medicament for treating cancer.
